**Europäisches Patentamt**

**European Patent Office**

**Office européen des brevets**

(19)

(11) Veröffentlichungsnummer: **0 022 488**
**B1**

# EUROPÄISCHE PATENTSCHRIFT

(12)

(45) Veröffentlichungstag der Patentschrift:
**20.01.82**

(21) Anmeldenummer: **80103507.2**

(22) Anmeldetag: **23.06.80**

(51) Int. Cl.³: **C 07 C 69/16,** C 07 C 67/283

(54) Verfahren zur Herstellung von Butandioldiacetat durch katalytische Hydrierung von Butendioldiacetat.

(30) Priorität: **13.07.79 DE 2928310**

(43) Veröffentlichungstag der Anmeldung:
**21.01.81 Patentblatt 81/3**

(45) Bekanntmachung des Hinweises auf die Patenterteilung:
**20.01.82 Patentblatt 82/3**

(84) Benannte Vertragsstaaten:
**BE CH DE FR GB IT LI NL**

(56) Entgegenhaltungen:
**DE-A1-2 513 522**
**US-A-4 164 616**

(73) Patentinhaber: **BASF Aktiengesellschaft,**
**Carl-Bosch-Strasse 38, D-6700 Ludwigshafen (DE)**

(72) Erfinder: **Broecker, Franz Josef, Dr., Schwanthaler**
**Alee 20, D-6700 Ludwigshafen (DE)**
Erfinder: **Platz, Rolf, Dr., Hansastrasse 5,**
**D-6800 Mannheim 1 (DE)**
Erfinder: **Schnabel, Rolf, Dr., Tilsiter Strasse 9,**
**D-6707 Schifferstadt (DE)**
Erfinder: **Stabenow, Joachim, Dr., Am Feldrain 22,**
**D-6940 Weinheim (DE)**
Erfinder: **Weitz, Hans-Martin, Dr., Auf dem Koeppel 40,**
**D-6702 Bad Duerkheim 1 (DE)**

EP 0 022 488 B1

Verfahren zur Herstellung von Butandioldiacetat durch katalytische Hydrierung von Butendioldiacetat

Diese Erfindung betrifft ein Verfahren zur Herstellung von Butandioldiacetat durch katalytische Hydrierung von Butendioldiacetat in der Gasphase.

Aus der DE-OS 27 23 961 ist bekannt, dass man Butandioldiacetat durch Umsetzung von Butadien mit Essigsäure und Sauerstoff in Gegenwart von Palladiumkatalysatoren und anschliessende Hydrierung des dabei erhältlichen Butendioldiacetates herstellen kann. Die Hydrierung wird in der Flüssigphase, vorzugsweise bei Drucken von 10 bis 200 bar durchgeführt. Auch nach dem in der DE-OS 23 45 160 beschriebenen Verfahren, bei dem man Nickel, Zink und/oder Vanadium enthaltende Katalysatoren verwendet, wird in der Flüssigphase hydriert.

Bei der technischen Durchführung dieser Hydrierungen in der Flüssigphase haben sich dadurch Schwierigkeiten ergeben, dass Nebenprodukte entstehen. Um die Menge an Nebenprodukten möglichst gering zu halten, ist man bemüht, bei sehr niedrigen Temperaturen zu hydrieren. Dieses hat zur Folge, dass die Katalysatoraktivität zur quantitativen Hydrierung nicht ausreicht. Bei einem technischen Verfahren ist man aber auf eine möglichst quantitative Hydrierung angewiesen, da eine destillative Trennung von Butandioldiacetat und Butendioldiacetat aufgrund der fast gleichen Siedetemperatur dieser Stoffe sehr aufwendig ist. Man hat deshalb in der DE-OS 23 37 890 ein zweistufiges Hydrierverfahren vorgeschlagen, bei dem das Reaktionsgemisch zwei Reaktionszonen mit unterschiedlichen Temperatur- und Druckbereichen zugeleitet wird. Dieses Verfahren ist umständlich und technisch sehr aufwendig.

Da Butandioldiacetat als wertvolles Zwischenprodukt z. B. für die Herstellung von Tetrahydrofuran zunehmende technische Bedeutung erlangt hat, war nach einem Verfahren zu suchen, das es gestattet, die Hydrierung von Butendioldiacetat auf Butandioldiacetat auf möglichst einfache Weise und nahezu quantitativ durchzuführen.

Nach dem erfindungsgemässen Verfahren, durch das diese Aufgabe gelöst wird, erhält man Butandioldiacetat durch katalytische Hydrierung von Butendioldiacetat, indem man die Hydrierung in der Gasphase durchführt.

Überraschenderweise erhält man bei dem neuen einstufigen Verfahren im Gegensatz zur Flüssigphasenhydrierung extrem gute Selektivitäten bei sehr weitgehender Hydrierung der Doppelbindung.

Als Butendioldiacetat setzt man z. B. solche Butendioldiacetat enthaltende Gemische ein, die man auf an sich bekannte Weise durch katalytische Umsetzung von 1,3-Butadien, Essigsäure und Sauerstoff erhält und die neben Butendioldiacetat-1,4 und Butendioldiacetat-1,2 geringe Mengen an Butandioldiacetat-1,4, Butandioldiacetat-1,2, Butendiolmonoacetate, Butendiol und Essigsäure enthalten können.

Die zu hydrierenden Butendioldiacetate werden zweckmässig vor Eintritt in den Hydrierreaktor verdampft. Man führt die Hydrierung in der Gasphase, z. B. bei Temperaturen bis 150 °C, vorzugsweise bei 80 bis 120 °C durch. Die Partialdrüke des Butendioldiacetates, die man über die Verdampfertemperatur einstellen kann, betragen in der Regel bis zu 200 mbar; sie liegen vorzugsweise zwischen 5 und 100 mbar. Als Katalysatoren lassen sich die an sich üblichen Hydrierkatalysatoren verwenden. Beispielsweise verwendet man Katalysatoren, die als Aktivkomponenten Metalle der 8. Nebengruppe enthalten, vorzugsweise Nickel- und Palladiumkatalysatoren. Den Wasserstoffpartialdruck stellt man zweckmässigerweise mindestens so hoch wie den Butendioldiacetatpartialdruck ein. Nach oben hin ist der Wasserstoffdruck nicht begrenzt. Ein Zusatz von Inertgasen, wie Methan oder Stickstoff ist möglich.

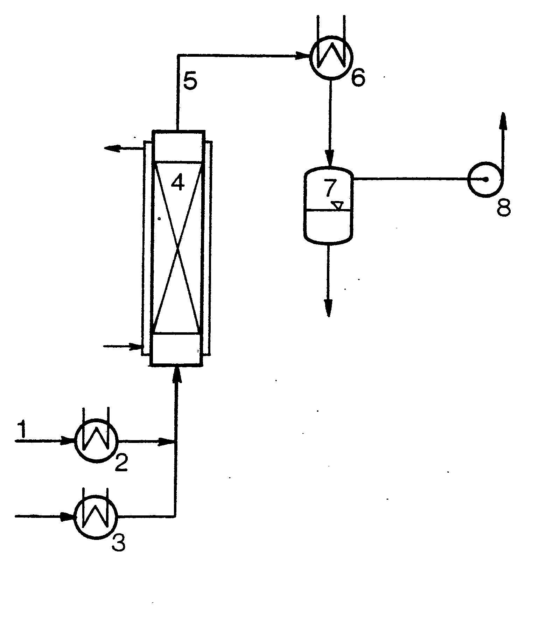

Nach einer vorteilhaften Ausführungsform der Erfindung führt man die Hydrierung in der durch die Abbildung veranschaulichten Apparatur durch.

Das zu hydrierende Butendioldiacetat wird durch die Zuleitung (1) in einen Verdampfer (2) geleitet, in dem es verdampft wird. Der gasförmige Ausgangsstoff wird zusammen mit dem im Gaserhitzer (3) aufgeheizten Wasserstoff in einen Hydrierreaktor (4) geleitet. Der mit dem Katalysator beschickte Reaktor ist mit einer Beheizung über einen Thermostaten versehen. Das gasförmige Reaktionsgemisch gelangt über die Ableitung (5) in einen Kondensator (6) in dem das Butandioldiacetat kondensiert wird, welches dann im Abscheider (7) gesammelt wird. Der überschüssige Wasserstoff wird mit einer Vakuumpumpe (8) abgeführt. Er kann z. B. dem Gaserhitzer (3) zur erneuten Verwendung in dem Hydrierverfahren zugeführt werden.

Beispiel 1

Der aus der Abbildung ersichtliche Hydrierreaktor wird mit einem Palladium/Kohle-Katalysator (0,2 Gewichtsprozent Pd) beschickt. Aus einem Vorratsbehälter wird ein Butendioldiacetatgemisch in den Verdampfer gepumpt. Das Butendioldiacetatgemisch hat folgende Zusammensetzung in Gewichtsprozent:

94,89 Gew.% cis-Butendioldiacetat 1,4
6,56 Gew.% trans-Butendioldiacetat 1,4
0,43 Gew.% Butandioldiacetat-1,4
0,54 Gew.% Essigsäure
0,54 Gew.% Butandioldiacetat-1,2

Die Temperatur im Verdampfer und im Reaktor wird auf 100 °C gehalten. Mittels eines Regulierventils werden 25 Nl/h Wasserstoff zudosiert. Man reguliert die Vakuumpumpe so, dass sich in der Apparatur ein Gesamtdruck von 20 mbar einstellt. Die Belastung beträgt 0,5 kg/1$_{Kat.}$·h. Das den Hydrierreaktor verlassende Produkt wird kondensiert und analysiert. Gaschromatographisch wird folgende Zusammensetzung ermittelt:

1,43 Gew.% cis-Butendioldiacetat-1,4
0,59 Gew.% trans-Butendioldiacetat-1,4
97,33 Gew.% Butandioldiacetat-1,4
0,24 Gew.% Butylacetat
0,05 Gew.% Essigsäure
0,31 Gew.% Butandioldiacetat-1,2

Beispiel 2

Man verfährt wie im Beispiel 1 beschrieben, wobei man jedoch als Katalysator einen Ni/SiO$_2$-Katalysator verwendet, der durch Fällen einer Wasserglas, Nickelnitrat und Salpetersäure enthaltenden wässrigen Lösung mit einem pH-Wert von 2 durch eine 2-molare wässrige Natriumibicarbonatlösung bei 60°C, Filtration, Trocknen bei 120°C, Kalzinieren bei 350°C und Verformung des Pulvers zu Tabletten hergestellt und nach Einbau in den Reaktor bei 400°C mit Wasserstoff reduziert wurde. Das Hydrierprodukt hat folgende prozentuale Zusammensetzung:

3,91 Gew.% cis-Butendioldiacetat-1,4
3,92 Gew.% trans-Butendioldiacetat-1,4
90,77 Gew.% Butandioldiacetat-1,4
0,81 Gew.% Butylacetat
0,19 Gew.% Essigsäure
0,40 Gew.% Butandioldiacetat-1,2

Vergleichsbeispiel a)

Von dem im Beispiel 1 verwendeten Pd/Kohle-Katalysator werden 200 ml in einem Kreislaufhydrierreaktor eingebaut und mit dem in Beispiel 1 verwendeten Butendioldiacetatgemisch beaufschlagt. Man hydriert gemäss der Flüssigfahrweise unter folgenden Bedingungen: Temperatur 100°C, Druck 30 bar, Belastung 0,5 kg/1$_{Kat.}$·h und Querschnittsbelastung

$$50 \ \frac{m^3}{m^2 \cdot h} \ \cdot$$

Das Hydrierprodukt hat folgende Zusammensetzung:

91,5% cis-Butendioldiacetat-1,4
6,5% trans-Butendioldiacetat-1,4
0,5% Butandioldiacetat-1,4
0,2% Essigsäure
0,7% Butylacetat
0,5% Butandioldiacetat-1,2

Vergleichsbeispiele b)

Man hydriert wie im Vergleichsbeispiel a) beschrieben, jedoch bei einer Temperatur von 150°C und einem Druck von 100 bar. Das Hydrierprodukt hat folgende Zusammensetzung:

12,4% cis-Butendioldiacetat-1,4
6,3% trans-Butendioldiacetat-1,4
32,9% Butandioldiacetat-1,4
16,4% Essigsäure
27,5% Butylacetat
2,2% Butandioldiacetat-1,2
Zeichn.

**Patentansprüche**

1. Verfahren zur Herstellung von Butandioldiacetat durch katalytische Hydrierung von Butendioldiacetat, dadurch gekennzeichnet, dass man die Hydrierung in der Gasphase durchführt.

2. Verfahren nach Anspruch 1, dadurch gekennzeichnet, dass man die Hydrierung bei Temperaturen bis 150°C und bei Partialdrucken des Butendioldiacetats bis zu 200 mbar durchführt.

3. Verfahren nach Anspruch 1, dadurch gekennzeichnet, dass man die Hydrierung bei Temperaturen von 80 bis 120°C und bei Partialdrucken des Butendioldiacetats von 5 bis 100 mbar durchführt.

**Claims**

1. A process for the preparation of butanediol diacetate by catalytic hydrogenation of butenediol diacetate, characterized in that the hydrogenation is carried out in the gas phase.

2. A process as claimed in claim 1, characterized in that the hydrogenation is carried out at up to 150°C with a partial pressure of butenediol diacetate of up to 200 mbar.

3. A process as claimed in claim 1, characterized in that the hydrogenation is carried out at from 80 to 120°C with a partial pressure of butenediol diacetate of from 5 to 100 mbar.

**Revendications**

1. Procédé pour la préparation de diacétate de butane-diol par hydrogénation catalytique de diacétate de butène-diol, caractérisé en ce qu'on effectue l'hydrogénation en phase gazeuse.

2. Procédé selon la revendication 1, caractérisé en ce qu'on effectue l'hydrogénation à une température pouvant aller jusqu'à 150°C et sous une pression partielle du diacétate de butène-diol qui peut aller jusqu'à 200 mbar.

3. Procédé selon la revendication 1, caractérisé en ce qu'on effectue l'hydrogénation à une température comprise entre 80 et 120°C et sous une pression partielle du diacétate de butène-diol comprise entre 5 et 100 mbar.